# EUROPEAN PATENT SPECIFICATION

(11) **EP 0 745 396 B1**
(45) Date of publication and mention of the grant of the patent: **04.02.2004**
(21) Application number: 96500066.4
(22) Date of filing: 28.05.1996
(51) Int. Cl.: A61M 1/10

(54) **Blood pump and application thereof**
Blutpumpe und ihre Anwendung
Pompe sanguine et son application

(30) Priority: 30.05.1995 ES 9501070
(43) Date of publication of application: 04.12.1996
(73) Proprietor: SERVICIO REGIONAL DE SALUD, DE LA CONSEJERIA DE SALUD DE LA COMUNIDAD DE MADRID, 28009 Madrid (ES)
(72) Inventor: del Canizo Lopez, Juan Francisco, 28006 Madrid (ES)
(74) Representative: Elzaburu Marquez, Alberto

(56) References cited:
- WO-A-94/27657
- US-A- 3 007 416
- US-A- 4 047 844
- US-A- 4 552 552
- US-A- 4 650 457

## Description

The present invention refers to a blood pumping system, tubular in shape, governed by vacuum, in which propulsion direction is obtained by stenosizers of external tube and which is actuated by a console that controls all its functions.

### FIELD OF THE INVENTION

This invention may be applied within the industry dedicated to the manufacture of medicinal apparatus.

### BACKGROUND OF THE INVENTION

There are several situations in medicine where it is necessary to pump blood, by means of an artificial device.
1.- During open heart surgery by means of what is called the extracorporeal circulation (ECC).
2.- In acute cardiac or respiratory failure in which the so-called extracorporeal membrane oxygenator is used (ECMO).
3.- In patients with profound ventricular failure in which the so-called artificial ventricles may be used.
4.- In Hemodialysis with the so-called artificial kidney and in the new apheresis systems.

All these cases need to use a device which pumps blood, with the following features:
1.- Be effective from a hemodynamic point of view, i.e they must be capable of supplying an appropriate blood flow in all the conditions which may arise within the specific application.
2.- The mechanical effort which the blood forming elements are submitted to, especially red globules, must be minimal, i.e. homolysis must not arise.
3.- They must not produce any formation of added plaques nor thrombi which may be released and lead to embolies in the patient.

Optimization of the blood pumps depend on four types of solutions.
A.- Solutions which help to reduce the formation of added plaques, basically aimed at being designed so as to avoid areas of blood stagnation.
B.- Solutions aimed at reducing the lesions produced in the blood forming elements.
C.- Solutions aimed at reducing the cost of the device in order to extend its application field.
D.- In some applications such as the use of artificial ventricules there must be solutions to improve the filling up of the blood chamber.

The device presented offers new solutions in these four aspects. It many be defined as a blood pump, of tubular shape, actuated by vacuum in which the propulsion direction is achieved by stenosizers of active external tube (also called pincer valves) instead of the classical valves and which is actuated by a console which controls alls its functions. In some applications, especially those related to artificial ventricles, a second, complementary chamber may be added to optimize filling, at the pump entrance.

The majority of blood pump devices in the art today are based on the compression of a chamber, either by means of a gas, or by means of rollers in order to reduce the dimensions and provoke liquid propulsion. In this device a new concept is used. The propulsion chamber is an elastic tube with thin walls which are found on the inside of a rigid chamber in which it is possible to exercise vacuum thanks to a connection with the command console. Similar tubular shaped membrane blood pumping systems, as the one describe herein, have been disclosed in US 3007416 and US 4552552. However those prior art blood pump are actuated by the combination of gauge or positive pressure and vacuum, wherein blood is propulsed out by the action of the gauge or positive pressure that collapses the tubular shaped. In the invention the vacuum applied to the tubular chamber makes this dilate homogeneously without any wrinkles which could encourage the formation of added plaques and clots. Moreover, the actual tubular shape of the chamber is favourable to this circumstance. When the vacuum is interrupted and the rigid chamber is connected to atmospheric pressure the elastic recuperation of the membrane provokes blood propulsion.

Use of the vacuum also offers another advantage, if the membrane should perforate, gas will never enter the inside of the blood chamber because the pressure in the same is always higher than that in the external chamber, avoiding the possibility of gaseous embolies in the patient.

Use of active, external stenosizers dramatically reduces the cost of the device compared with those which use valves, since the valves used for these applications are very expensive and make the cost of these devices prohibitive, especially in applications such as hemodialysis or extracorporeal circulation. Moreover, use of stenosizers means that the entrance and exit diameters of the pump nozzles are completely uniform and adaptable to any type of application since the smallest valves existing on the market are around 16 mm diameter although in some applications such as pediatrics or dialysis, the nozzles used are of much lower diameter.

Lesions of formative blood elements are minimized in this device since the only source of lesion would be from closing the stenosizers which is only done in two specific points, compared with the roller pump which continuously squeezes the content of the tube and contrary to classical values which, when open, always have some surface area over which the blood has to circulate. In the solution proposed, when the stenosizer opens, the tube section is completely homogeneous.

In some applications, above all those in which it is necessary to control high flows, as in the use of artificial ventricles, it may be necessary to add a complementary chamber in the entrance nozzle to encourage filling. This chamber would be the same type as the propulsion chamber, in such a way that its compliance could be modified, varying the vacuum level applied to the same, depending on its operation conditions.

In this type of applications safety is also an essential factor.

The design of this pump allows for connecting two in parallel form so that one of them acts as a security mechanism with their two stenosizers closed whilst inactive, being able to automatically operate should the need arise if the main pump fails.

The design of the pump also allows for fluid inversion, simply inverting the sequence of aperture of the stenosizers, which is not possible in those devices which use passive mechanical valves.

### DESCRIPTION

The present application refers to a tubular shaped blood pump, actuated by vacuum in which the propulsion direction is obtained by stenosizers of active external tubes instead of valves and which is actuated by a console to control all its functions.

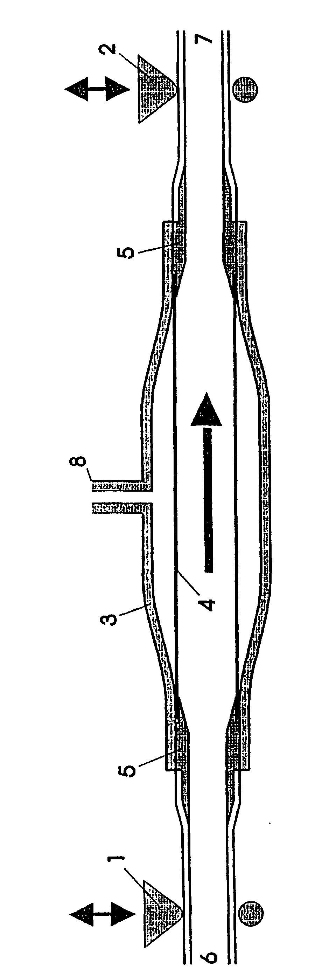

The single drawing represents a transversal section of the device.
1.- Entrance stenosizers
2.- Exit stenosizers
3.- Rigid chamber
4.- Elastic tubular membrane
5.- Nozzle connection pieces
6.- Elastic entrance nozzle
7.- Elastic exit nozzle
8.- Connection for vacuum

### Operation

The connection for the vacuum connects the sealed space which exists between the membrane and the chamber with the command console (Single drawing).

When the pump is filling up, the command console carries out a suction through the connection (8) provoking the expansion of the membrane (4), at the same time as the closure of the exit stenosizor order (2), and the opening of the entrance (1) resulting in the blood penetrating through the entrance nozzle filling the chamber.

During propulsion, the command console connects the vacuum connection (8) with the atmosphere, at the same time as giving the order to close the entrance stenosizer (1) and to open the exit (2), at which time the elastic recuperation of the membrane (4), freed from the vacuum exercised during filling up time, propulses the blood through the exit nozzle (7).

## Claims

1. A tubular shaped blood pump device using vacuum as the main source of energy, comprising entrance (6) and exit (7) nozzles, active valves adapted to work as active external tube stenosizers (1,2) and situated at the entrance (6) and the exit (7) nozzles, a tubular elastic membrane having an initial shape with an homogeneous section throughout the length of the membrane located inside of a rigid chamber (3), a command console for both supplying the necessary energy and controlling the pump, the command console being adapted to close the exit stenosizer (2), to open the entrance stenosizer (1) and to connect the rigid chamber (3) to vacuum, said vacuum dilates the tubular elastic membrane in order to allow blood intake, **characterized in that** the command console is adapted to close the entrance stenosizer (1), to open the exit stenosizer (2), to disconnect the rigid chamber from the vacuum and to connect said chamber to the atmosphere, whereby blood is propulsed out through the exist nozzle only by the elastic recuperation of said tubular membrane when said chamber is connected to the atmosphere.

2. Tubular shaped blood pump device, according to previous claim 1, **characterized in that** the tube stenosizers means (1,2) used, may be pneumatic, electric, electromagnetic or mechanical.

3. Tubular shaped blood pump device, according to the above claims 1 and 2, **characterized by** possessing a command console which supplies the necessary energy, commands connection and disconnection of vacuum, commands opening and closure of stenosizers in precise moments of the cycle.

4. Use of the tubular shaped blood pump device, of claims 1 to 3, as a pump unit in hemodialysis, hemofiltration and apheresis systems.

5. Use of the tubular shaped blood pump device, of claims 1 to 3, as a pump unit in oxygenation systems by extracorporal membrane.

6. Use of the tubular shaped blood pump device, of claims 1 to 3, as a pump in extracorporal circulation systems.

7. Use of the tubular shaped blood pump device, of claims 1 to 3 as an artificial ventricle in systems of mechanical circulatory assistance.

8. Use according to claims 4 to 7, of at least two tubular shaped blood pump devices according to claims 1 to 3 connected in parallel.

## Patentansprüche

1. Röhrenförmig geformte Blutpumpenvorrichtung, welche als Hauptenergiequelle ein Vakuum verwendet, aufweisend Eingangs-(6) und Ausgangs-(7)-Düsen, aktive Ventile, welche angepasst sind, um als aktive, äußere Röhrenstenisatoren (1, 2) zu arbeiten, und welche an den Eingangs-(6) und Ausgangs-(7)-Düsen sich befinden, eine röhrenförmige, elastische Membran, welche eine anfängliche Form mit einem homogenen Abschnitt über die Länge der Membran aufweist, angeordnet im Inneren einer festen Kammer (3), eine Kommandokonsole sowohl zur Zufuhr der nötigen Energie und zur Steuerung-der Pumpe, wobei die Kommandokonsole angepasst ist, um den Ausgangsstenisator (2) zu schließen, den Eingangsstenisator (1) zu öffnen und die feste Kammer (3) mit einem Vakuum zu verbinden, wobei das Vakuum die röhrenförmige elastische Membran aufweitet, um einen Bluteinlass zu erlauben, **dadurch gekennzeichnet, dass** die Kommandokonsole angepasst ist, um den Eingangsstenisator (1) zu schließen, den Ausgangsstenisator (2) zu öffnen, die feste Kammer von dem Vakuum abzukoppeln und die Kammer mit der Atmosphäre zu verbinden, wobei Blut durch das Auslassventil nur durch die elastische Rückbewegung der röhrenförmigen Membran herausgetrieben wird, wenn die Kammer mit der Atmosphäre verbunden ist.

2. Röhrenförmig geformte Blutpumpenvorrichtung gemäß dem vorherigen Anspruch 1, **dadurch gekennzeichnet, dass** die verwendeten Röhrenstenisatormittel (1, 2) pneumatisch, elektrisch, elektromagnetisch oder mechanisch sein können.

3. Röhrenförmig geformte Blutpumpenvorrichtung gemäß den obigen Ansprüchen 1 und 2, **dadurch gekennzeichnet, dass** sie eine Kommandokonsole besitzt, welche die nötige Energie zuführt, die Verbindung und Trennung des Vakuums befehligt, und ein Öffnen und Schließen der Stenisätoren in exakten Augenblicken des Zyklus befehligt.

4. Verwendung der röhrenförmig geformten Blutpumpenvorrichtung der Ansprüche 1 bis 3 als eine Pumpeneinheit in Hämodialyse-, Hämofiltrationund Apheresesystemen.

5. Verwendung der röhrenförmig geformten Blutpumpenvorrichtung der Ansprüche 1 bis 3 als Pumpeneinheit in Sauerstoffanlagerungssystemen durch extrakorporale Membrane.

6. Verwendung der röhrenförmig geformten Blutpumpenvorrichtung der Ansprüche 1 bis 3 als Pumpe in extrakorporalen Zirkulationssystemen.

7. Verwendung der röhrenförmig geformten Blutpumpenvorrichtung der Ansprüche 1 bis 3 als künstliches Ventrikel in Systemen mechanischer Zirkulationsunterstützung.

8. Verwendung gemäß den Ansprüchen 4 bis 7 von zumindest zwei röhrenförmig geformten Blutpumpenvorrichtungen gemäß den Ansprüchen 1 bis 3, welche parallel verbunden sind.

## Revendications

1. Dispositif à pompe à sang de forme tubulaire utilisant une dépression en tant que source principale d'énergie, comportant des buses d'entrée (6) et de sortie (7), des soupapes actives conçues pour travailler en tant qu'organes de rétrécissement de tube extérieur actif (1, 2) et situées aux buses d'entrée (6) et de sortie (7), une membrane élastique tubulaire ayant une forme initiale présentant une section homogène sur toute la longueur de la membrane située à l'intérieur d'une chambre rigide (3), une console de commande destinée à la fois à fournir l'énergie nécessaire et à commander la pompe, la console de commande étant conçue pour fermer l'organe de rétrécissement de sortie (6), ouvrir l'organe de rétrécissement d'entrée (1) et raccorder la chambre rigide (3) à une dépression, ladite dépression dilate la membrane élastique tubulaire afin de permettre l'admission de sang, **caractérisé en ce que** la console de commande est conçue pour fermer l'organe de rétrécissement d'entrée (1), pour ouvrir l'organe de rétrécissement de sortie (2), pour isoler la chambre rigide de la dépression et pour raccorder ladite chambre à l'atmosphère, grâce à quoi du sang est propulsé vers l'extérieur à travers la buse de sortie uniquement par le retour élastique de ladite membrane tubulaire lorsque ladite chambre est raccordée à l'atmosphère.

2. Dispositif à pompe à sang de forme tubulaire selon la revendication précédente 1, **caractérisé en ce que** les organes de rétrécissement (1, 2) à tubes utilisés peuvent être pneumatiques, électriques, électromagnétiques ou mécaniques.

3. Dispositif à pompe à sang de forme tubulaire selon les revendications 1 et 2 ci-dessus, **caractérisé en ce qu'**il comporte une console de commande qui fournit l'énergie nécessaire, commande le raccordement et la coupure de la dépression, commande l'ouverture et la fermeture des organes de rétrécissement à des moments précis du cycle.

4. Utilisation du dispositif à pompe à sang de forme tubulaire selon les revendications 1 à 3, en tant qu'unité de pompe dans des systèmes d'hémodialyse, d'hémofiltration et d'aphérèse.

5. Utilisation du dispositif à pompe à sang de forme tubulaire selon les revendications 1 à 3, en tant qu'unité de pompage dans des systèmes d'oxygénation par membrane extracorporelle.

6. Utilisation du dispositif à pompe à sang de forme tubulaire selon les revendications 1 à 3, en tant que pompe dans des systèmes de circulation extracorporelle.

7. Utilisation du dispositif à pompe à sang de forme tubulaire selon les revendications 1 à 3, en tant que ventricule artificiel dans des systèmes d'assistance circulatoire mécanique.

8. Utilisation selon les revendications 4 à 7 d'au moins deux dispositifs à pompes à sang de forme tubulaire selon les revendications 1 à 3, raccordés en parallèle.
